# EUROPEAN PATENT APPLICATION

(11) **EP 0 962 530 A2**
(43) Date of publication of application: **08.12.1999**
(21) Application number: 99304239.9
(22) Date of filing: 01.06.1999
(51) Int. Cl.: C12N 15/12, C07K 14/515, C12N 15/63, C12N 5/10, A61K 38/18, G01N 33/50

(54) **Gene sequence encoding for the angiopoietin related protein scarface 1**

(30) Priority: 02.06.1998 US 87710 P; 08.06.1998 US 88503 P; 17.09.1998 US 100857 P
(71) Applicant: ELI LILLY AND COMPANY, Indianapolis, Indiana 46285 (US)
(72) Inventor: Heuer, Josef Georg, Indianapolis, Indiana 46237 (US); Burgett, Stanley Gene, Indianapolis, Indiana 46227 (US); Leonard, Rebecca Ann, Indianapolis, Indiana 46260 (US); Rosteck, Paul Robert, Jr., Indianapolis, Indiana 46237 (US); Sankhavaram, Patanjali Raghavacharya, Carmel, Indiana 46033 (US)
(74) Representative: Denholm, Anna Marie

(57) **Abstract**

The invention provides angiopoietin related sequence Scarface 1, analogs thereof, and isolated nucleic acid compounds encoding same. Also provided are pharmaceutical compositions, vectors and transformed heterologous host cells for expressing the Scarface 1 protein, and a method for identifying compounds that bind and/or modulate the activity of said protein.

### Annex to the description

SEQUENCE LISTING

<110> Burgett, Stanley G.
      Little, Rebecca A.
      Rosteck Jr., Paul R.
      Heuer, Josef G.
      Sankhavaram, Patanjali R.

<120> Angiopoietin Related Gene Sequence Scarface 1

<130> X-12260

<140>
<141>

<160> 2

<170> PatentIn Ver. 2.0

<210> 1
<211> 1479
<212> DNA
<213> Homo sapiens

<220>
<221> CDS
<222> (1)..(1479)

<400> 1
atg agg cca ctg tgc gtg aca tgc tgg tgg ctc gga ctg ctg gct gcc    48
Met Arg Pro Leu Cys Val Thr Cys Trp Trp Leu Gly Leu Leu Ala Ala
  1               5                  10                  15

atg gga gct gtt gca ggc cag gag gac ggt ttt gag ggc act gag gag    96
Met Gly Ala Val Ala Gly Gln Glu Asp Gly Phe Glu Gly Thr Glu Glu
             20                  25                  30

ggc tcg cca aga gag ttc att tac cta aac agg tac aag cgg gcg ggc    144
Gly Ser Pro Arg Glu Phe Ile Tyr Leu Asn Arg Tyr Lys Arg Ala Gly
         35                  40                  45

gag tcc cag gac aag tgc acc tac acc ttc att gtg ccc cag cag cgg    192
Glu Ser Gln Asp Lys Cys Thr Tyr Thr Phe Ile Val Pro Gln Gln Arg
     50                  55                  60

gtc acg ggt gcc atc tgc gtc aac tcc aag gag cct gag gtg ctt ctg    240
Val Thr Gly Ala Ile Cys Val Asn Ser Lys Glu Pro Glu Val Leu Leu
 65                  70                  75                  80

gag aac cga gtg cat aag cag gag cta gag ctg ctc aac aat gag ctg    288
Glu Asn Arg Val His Lys Gln Glu Leu Glu Leu Leu Asn Asn Glu Leu
                 85                  90                  95

ctc aag cag aag cgg cag atc gag acg ctg cag cag ctg gtg gag gtg    336
Leu Lys Gln Lys Arg Gln Ile Glu Thr Leu Gln Gln Leu Val Glu Val
            100                 105                 110

gac ggc ggc att gtg agc gag gtg aag ctg ctg cgc aag gag agc cgc    384

Asp Gly Gly Ile Val Ser Glu Val Lys Leu Leu Arg Lys Glu Ser Arg
        115                 120                 125

aac atg aac tcg cgg gtc acg cag ctc tac atg cag ctc ctg cac gag 432
Asn Met Asn Ser Arg Val Thr Gln Leu Tyr Met Gln Leu Leu His Glu
    130                 135                 140

atc atc cgc aag cgg gac aac gcg ttg gag ctc tcc cag ctg gag aac 480
Ile Ile Arg Lys Arg Asp Asn Ala Leu Glu Leu Ser Gln Leu Glu Asn
145                 150                 155                 160

agg atc ctg aac cag aca gcc gac atg ctg cag ctg gcc agc aag tac 528
Arg Ile Leu Asn Gln Thr Ala Asp Met Leu Gln Leu Ala Ser Lys Tyr
                165                 170                 175

aag gac ctg gag cac aag tac cag cac ctg gcc aca ctg gcc cac aac 576
Lys Asp Leu Glu His Lys Tyr Gln His Leu Ala Thr Leu Ala His Asn
            180                 185                 190

caa tca gag atc atc gcg cag ctt gag gag cac tgc cag agg gtg ccc 624
Gln Ser Glu Ile Ile Ala Gln Leu Glu Glu His Cys Gln Arg Val Pro
        195                 200                 205

tcg gcc agg ccc gtc ccc cag cca ccc ccc gct gcc ccg ccc cgg gtc 672
Ser Ala Arg Pro Val Pro Gln Pro Pro Pro Ala Ala Pro Pro Arg Val
    210                 215                 220

tac caa cca ccc acc tac aac cgc atc atc aac cag atc tct acc aac 720
Tyr Gln Pro Pro Thr Tyr Asn Arg Ile Ile Asn Gln Ile Ser Thr Asn
225                 230                 235                 240

gag atc cag agt gac cag aac ctg aag gtg ctg cca ccc cct ctg ccc 768
Glu Ile Gln Ser Asp Gln Asn Leu Lys Val Leu Pro Pro Pro Leu Pro
245 . 250 255

act atg ccc act ctc acc agc ctc cca tct tcc acc gac aag ccg tcg 816
Thr Met Pro Thr Leu Thr Ser Leu Pro Ser Ser Thr Asp Lys Pro Ser
260 265 270

ggc cca tgg aga gac tgc ctg cag gcc ctg gag gat ggc cac gac acc 864
Gly Pro Trp Arg Asp Cys Leu Gln Ala Leu Glu Asp Gly His Asp Thr
275 280 285

agc tcc atc tac ctg gtg aag ccg gag aac acc aac cgc ctc atg cag 912
Ser Ser Ile Tyr Leu Val Lys Pro Glu Asn Thr Asn Arg Leu Met Gln
290 295 300

gtg tgg tgc gac cag aga cac gac ccc ggg ggc tgg acc gtc atc cag 960
Val Trp Cys Asp Gln Arg His Asp Pro Gly Gly Trp Thr Val Ile Gln
305 310 315 320

aga cgc ctg gat ggc tct gtt aac ttc ttc agg aac tgg gag acg tac
1008
Arg Arg Leu Asp Gly Ser Val Asn Phe Phe Arg Asn Trp Glu Thr Tyr
325 330 335

aag caa ggg ttt ggg aac att gac ggc gaa tac tgg ctg ggc ctg gag
1056

Lys Gln Gly Phe Gly Asn Ile Asp Gly Glu Tyr Trp Leu Gly Leu Glu
340 345 350

aac att tac tgg ctg acg aac caa ggc aac tac aaa ctc ctg gtg acc
1104
Asn Ile Tyr Trp Leu Thr Asn Gln Gly Asn Tyr Lys Leu Leu Val Thr
355 360 365

atg gag gac tgg tcc ggc cgc aaa gtc ttt gca gaa tac gcc agt ttc
1152
Met Glu Asp Trp Ser Gly Arg Lys Val Phe Ala Glu Tyr Ala Ser Phe
370 375 380

cgc ctg gaa cct gag agc gag tat tat aag ctg cgg ctg ggg cgc tac
1200
Arg Leu Glu Pro Glu Ser Glu Tyr Tyr Lys Leu Arg Leu Gly Arg Tyr
385 390 395 400

cat ggc aat gcg ggt gac tcc ttt aca tgg cac aac ggc aag cag ttc
1248
His Gly Asn Ala Gly Asp Ser Phe Thr Trp His Asn Gly Lys Gln Phe
405 410 415

acc acc ctg gac aga gat cat gat gtc tac aca gga aac tgt gcc cac
1296
Thr Thr Leu Asp Arg Asp His Asp Val Tyr Thr Gly Asn Cys Ala His
420 425 430

tac cag aag gga ggc tgg tgg tat aac gcc tgt gcc cac tcc aac ctc
1344
Tyr Gln Lys Gly Gly Trp Trp Tyr Asn Ala Cys Ala His Ser Asn Leu
435 440 445

aac ggg gtc tgg tac cgc ggg ggc cat tac cgg agc cgc tac cag gac
1392
Asn Gly Val Trp Tyr Arg Gly Gly His Tyr Arg Ser Arg Tyr Gln Asp
450 455 460

gga gtc tac tgg gct gag ttc cga gga ggc tct tac tca ctc aag aaa
1440
Gly Val Tyr Trp Ala Glu Phe Arg Gly Gly Ser Tyr Ser Leu Lys Lys
465 470 475 480

gtg gtg atg atg atc cga ccg aac ccc aac acc ttc cac
1479
Val Val Met Met Ile Arg Pro Asn Pro Asn Thr Phe His
485 490

<210> 2
<211> 493
<212> PRT
<213> Homo sapiens

<400> 2
Met Arg Pro Leu Cys Val Thr Cys Trp Trp Leu Gly Leu Leu Ala Ala
1               5                   10                  15

Met Gly Ala Val Ala Gly Gln Glu Asp Gly Phe Glu Gly Thr Glu Glu
            20                  25                  30

Gly Ser Pro Arg Glu Phe Ile Tyr Leu Asn Arg Tyr Lys Arg Ala Gly
        35                  40                  45

Glu Ser Gln Asp Lys Cys Thr Tyr Thr Phe Ile Val Pro Gln Gln Arg
    50                  55                  60

Val Thr Gly Ala Ile Cys Val Asn Ser Lys Glu Pro Glu Val Leu Leu
65                  70                  75                  80

Glu Asn Arg Val His Lys Gln Glu Leu Glu Leu Leu Asn Asn Glu Leu
                85                  90                  95

Leu Lys Gln Lys Arg Gln Ile Glu Thr Leu Gln Gln Leu Val Glu Val
            100                 105                 110

Asp Gly Gly Ile Val Ser Glu Val Lys Leu Leu Arg Lys Glu Ser Arg
        115                 120                 125

Asn Met Asn Ser Arg Val Thr Gln Leu Tyr Met Gln Leu Leu His Glu
    130                 135                 140

Ile Ile Arg Lys Arg Asp Asn Ala Leu Glu Leu Ser Gln Leu Glu Asn
145                 150                 155                 160

Arg Ile Leu Asn Gln Thr Ala Asp Met Leu Gln Leu Ala Ser Lys Tyr
                165                 170                 175

Lys Asp Leu Glu His Lys Tyr Gln His Leu Ala Thr Leu Ala His Asn
            180                 185                 190

Gln Ser Glu Ile Ile Ala Gln Leu Glu Glu His Cys Gln Arg Val Pro
        195                 200                 205

Ser Ala Arg Pro Val Pro Gln Pro Pro Pro Ala Ala Pro Pro Arg Val
    210                 215                 220

Tyr Gln Pro Pro Thr Tyr Asn Arg Ile Ile Asn Gln Ile Ser Thr Asn
225                 230                 235                 240

Glu Ile Gln Ser Asp Gln Asn Leu Lys Val Leu Pro Pro Pro Leu Pro
                245                 250                 255

Thr Met Pro Thr Leu Thr Ser Leu Pro Ser Ser Thr Asp Lys Pro Ser
            260                 265                 270

Gly Pro Trp Arg Asp Cys Leu Gln Ala Leu Glu Asp Gly His Asp Thr
        275                 280                 285

Ser Ser Ile Tyr Leu Val Lys Pro Glu Asn Thr Asn Arg Leu Met Gln
    290                 295                 300

Val Trp Cys Asp Gln Arg His Asp Pro Gly Gly Trp Thr Val Ile Gln
305                 310                 315                 320

Arg Arg Leu Asp Gly Ser Val Asn Phe Phe Arg Asn Trp Glu Thr Tyr

                325                 330                 335

Lys Gln Gly Phe Gly Asn Ile Asp Gly Glu Tyr Trp Leu Gly Leu Glu
            340                 345                 350

Asn Ile Tyr Trp Leu Thr Asn Gln Gly Asn Tyr Lys Leu Leu Val Thr
        355                 360                 365

Met Glu Asp Trp Ser Gly Arg Lys Val Phe Ala Glu Tyr Ala Ser Phe
    370                 375                 380

Arg Leu Glu Pro Glu Ser Glu Tyr Tyr Lys Leu Arg Leu Gly Arg Tyr
385                 390                 395                 400

His Gly Asn Ala Gly Asp Ser Phe Thr Trp His Asn Gly Lys Gln Phe
                405                 410                 415

Thr Thr Leu Asp Arg Asp His Asp Val Tyr Thr Gly Asn Cys Ala His
            420                 425                 430

Tyr Gln Lys Gly Gly Trp Trp Tyr Asn Ala Cys Ala His Ser Asn Leu
        435                 440                 445

Asn Gly Val Trp Tyr Arg Gly Gly His Tyr Arg Ser Arg Tyr Gln Asp
    450                 455                 460

Gly Val Tyr Trp Ala Glu Phe Arg Gly Gly Ser Tyr Ser Leu Lys Lys
465                 470                 475                 480

Val Val Met Met Ile Arg Pro Asn Pro Asn Thr Phe His
                485                 490

## Description

### BACKGROUND OF THE INVENTION

This invention relates to recombinant DNA technology. In particular the invention pertains to angiogenesis related genes, their encoded proteins, and to pharmaceutical and other uses thereof.

Identifying the role of specific proteins in development and differentiation of higher organisms, including humans, has become a major challenge for modern molecular biology. In this regard the study of simpler organisms such as *Drosophila melanogaster* has greatly facilitated progress in this area. For example, a secreted protein termed "Scabrous" in *Drosophila,* plays an important role in defining positional aspects of developing neurogenic regions. Homology comparisons reveal that Scabrous is related to the angiopoietin family of proteins, which are necessary for normal angiogenesis in mammals.

Angiogenesis begins during embryonic development and continues throughout the life of the organism. During embroyonic development, a primitive vascular network forms by processes that involve regulated proliferation, differentiation, migration, and association of endothelial cells. Subsequent angiogenic processes remodel this primary network to form a mature cardiovascular system.

Angiogenic remodeling occurs throughout life in an adult organism, for example, during tissue repair and the menstrual cycle. Angiogenesis is also critically important during the proliferation and metastasis of tumor cells.

Mechanistically, angiogenesis involves multiple proteins, including tyrosine kinase receptors and protein ligands that bind the receptors to stimulate or inhibit angiogenesis. See e.g. J. Folkman and P. D'Amore, Cell 87, 1153-1155, 1996; S. Davies et al. Cell, 87, 1161-1169, 1996, herein incorporated by reference. The first protein to be identified in this process was dubbed "angiopoietin 1" (See S. Davis et al. Cell, 87, 1161-1169, 1996). Angiopoietin 1 comprises a 70 KD glycosylated protein of 498 amino acid residues in human and mouse. There are at least two recognizable domains on this protein, one a fibrinogen-like region at the carboxyl end, and the other a coiled-coil domain at the amino terminus. Coiled-coil regions typically are involved in the assembly of a protein into multimeric structures. It is thought that the coiled-coil region of angiopoietin 1 may serve to assemble the fibrinogen-like domain into a multimeric structure. The fibrinogen-like domain of angiopoietin 1 and other related molecules is probably the receptor binding portion for this class of molecules (See Valenzuela et al. Proc. Nat. Acad. Sci. 96, 1904-1909, 1999).

Angiopoietin 1 appears to function in angiogenesis by binding the Tie2 receptor and promoting tyrosine phosphorylation thereof. Interestingly, angiopoietin 1 does not stimulate the growth of endothelial cells in vitro, as does VEGF and fibroblast growth factor, for example. Thus, angiopoietin 1 would seem to play a role other than as a mitogen. The evidence to date favors a role in regulating the assembly of non-endothelial vessel wall components, and in controlling the maturation and stabilization of blood vessels (See C. Suri et al. Cell, 87, 1171-1180, 1996).

Angiopoietin 1 has a naturally occurring antagonist, termed angiopoietin 2, which blocks the ability of angiopoietin 1 to bind its receptor, Tie 2. Angiopoietin 2 appears to be expressed only at sites of vascular remodeling in the adult. (Maisonpierre et al. Science, 277, 55-60, 1997) The other receptor known to be required for normal angiogenesis is Tie1 *(See e.g.* D. Hanahan, *Science,* 277, 48, 1997). The Tie2 tyrosine kinase enables endothelial cells to recruit stromal cells for the encasement of endothelial tubes. Tiel, on the other hand, may be involved in the control of fluid exchange across capillary boundaries, and in hemodynamic stress resistance.

Recently, two additional angiopoietin-related proteins were identified in mouse and human, termed angiopoietin 3 and angiopoietin 4 (See Valenzuela et al. Proc. Nat. Acad. Sci. 96, 1904-1909, 1999). These molecules also bind the Tie2 receptor, angiopoietin 3 as an antagonist, but angiopoietin 4 as an agonist.

While the full complement of molecular players in the angiogenic process are yet to be defined, it is clear that additional angiopoietin-like ligands and receptors exist. The present invention relates to an angiopoietin-like gene and its encoded protein, as well as to pharmaceutical and other uses thereof.

### BRIEF SUMMARY OF THE INVENTION

The present invention provides angiopoietin-related nucleic acid molecules and their encoded proteins. The genes and proteins of the invention are referred to herein as "Scarface 1." A specific Scarface 1 gene sequence is disclosed herein as SEQ ID NO:1, which originated from a human source. The Scarface 1 nucleic acids of the invention are useful, among other things, as probes to isolate paralogous genes from humans and/or orthologous genes from other organisms.

The Scarface 1 peptides provide multiple utilities, for example, in screens to identify compounds that bind said peptides, as potential pharmaceutical compounds for modulating angiogenesis. Scarface 1 peptides are also useful as therapeutics to enhance wound healing, for developing compounds for inhibiting tumor growth, for treating cancer, and for use in a method to amplify stem cells.

The Scarface 1 proteins may further be useful in the diagnosis and treatment of certain diseases involving endothelial cells and associated TIE receptors, such as neoplastic diseases involving tumor angiogenesis, wound healing, thromboembolic diseases, atherosclerosis and inflammatory diseases. In addition, the mammalian ligand may be used to promote the proliferation and/or differentiation of hematopoietic stem cells.

More generally, biologically active mammalian Scarface 1 may be used to promote the growth, survival, migration, and/or differentiation and/or stabilization or destabilization of cells expressing TIE receptor. Biologically active Scarface 1 may be used for the in vitro maintenance of TIE receptor expressing cells in culture. Cells and tissues expressing TIE receptor include, for example, cardiac and vascular endothelial cells, lens epithelium and heart epicardium and early hematopoietic cells. Alternatively, Scarface 1 may be used to support cells which are engineered to express TIE receptor. Further, Scarface 1 and its cognate receptor may be used in assay systems to identify agonists or antagonists of TIE receptor.

The invention further provides a nucleic acid sequence encoding Scarface 1, and methods for the generation of nucleic acid encoding Scarface 1.

In one embodiment, the present invention relates to a substantially purified Scarface 1 peptide or fragment thereof.

In another embodiment, the present invention relates to a substantially purified protein preferably having an amino acid sequence that is at least 70% identical to SEQ ID NO:2, said protein having Scarface 1 activity in vivo or in vitro.

In another embodiment, the present invention relates to a substantially purified Scarface 1 peptide from a human source wherein said peptide has an amino acid sequence designated herein as SEQ ID NO:2.

In another embodiment, the present invention relates to a substantially purified protein having an amino acid sequence that is encoded by a nucleic acid sequence that hybridizes to SEQ ID NO:1 under high stringency conditions, said protein having Scarface 1 activity.

In another embodiment, the present invention relates to isolated nucleic acids encoding an Scarface 1 peptide.

In another embodiment, the present invention relates to isolated nucleic acids encoding Scarface 1, designated herein as SEQ ID NO:2.

In another embodiment, the present invention relates to an isolated nucleic acid sequence encoding Scarface 1 said nucleic acid being designated herein as SEQ ID NO:1.

In still another embodiment the present invention relates to a vector comprising an isolated nucleic acid of the present invention, wherein said isolated nucleic acid compound may be operably-linked to a promoter sequence.

In another embodiment, the present invention relates to a host cell containing a vector of the present invention.

In yet another embodiment, the present invention relates to a pharmaceutical formulation comprising as an active ingredient a therapeutically effective amount of an Scarface 1 peptide, associated with one or more pharmaceutically acceptable carriers, excipients, or diluents thereof.

In a further embodiment, the present invention relates to a pharmaceutical formulation comprising an Scarface 1 antagonist associated with one or more pharmaceutically acceptable carriers, excipients, or diluents thereof.

In a further embodiment, the present invention provides for an antibody which specifically binds Scarface 1. The antibody may be monoclonal or polyclonal.

The invention further provides for therapeutic compositions comprising an antibody which specifically binds Scarface 1 in a pharmaceutically acceptable vehicle.

In a further embodiment, the invention provides for a method of blocking blood vessel growth in a mammal by administering an effective amount of a therapeutic composition comprising an antibody which specifically binds Scarface 1 in a pharmaceutically acceptable vehicle.

The invention also provides for a method of promoting neovascularization in a patient by administering an effective amount of a therapeutic composition comprising Scarface 1 in a pharmaceutically acceptable vehicle. In one embodiment, the method may be used to promote wound healing. In another embodiment, the method may be used to treat ischemia. In yet another embodiment, Scarface 1 is used, alone or in combination with other hematopoietic factors, to promote the proliferation or differentiation of hematopoietic stem cells, B cells or megakaryocytic cells.

In still another embodiment the present invention relates to a method for identifying an antagonist of an Scarface 1 peptide, wherein said antagonist interferes in the binding of said Scarface 1 peptide to its receptor, comprising the steps of: mixing an Scarface 1 preparation with a test sample, or a control sample; monitoring the binding of said Scarface 1 peptide to its receptor, by any suitable means; and comparing the level of binding of Scarface 1 to its receptor in the test sample to the level of said binding in the control sample.

In yet another embodiment, the present invention relates to a method for suppressing tumor growth comprising the step of inhibiting the binding interaction in vivo of Scarface 1 to an Scarface 1 receptor by administering to a mammal in need thereof an effective amount of an Scarface 1 antagonist.

In another embodiment, the present invention relates to a method for stimulating angiogenesis by administration of an Scarface 1 peptide.

In still another embodiment, the present invention relates to a method for stimulating wound healing by administration of a therapeutically effective amount of an Scarface 1 peptide.

In still another embodiment, the present invention relates to a method to treat ischemia by administration of a therapeutically effective amount of an Scarface 1 peptide.

In another embodiment, the present invention relates to a method of blocking blood vessel growth in a mammal by administering an effective amount of a therapeutic composition comprising an antibody which specifically binds Scarface 1 in a pharmaceutically acceptable vehicle.

In still another embodiment, the invention provides for a method of blocking blood vessel growth in a mammal by administering an effective amount of a therapeutic composition comprising a receptorbody which specifically binds Scarface 1 in a pharmaceutically acceptable vehicle.

In still another embodiment, the present invention relates to an antibody that selectively binds to an Scarface 1 peptide.

In yet another embodiment, the present invention relates to a method for promoting the proliferation of, or expanding stem cells comprising the step of administration of an Scarface 1 peptide to a tissue or to a population of cells that contain said stem cells.

In still another embodiment, the present invention relates to a method for expanding hematopoietic stem cells comprising the step of administration of an Scarface 1 peptide to a population of bone marrow derived cells.

### DETAILED DESCRIPTION OF THE INVENTION

### Definitions

The term "analog" or "functional analog" refers to a related modified form of Scarface 1, in which at least one amino acid substitution, deletion, or addition has been made such that said analog retains substantially the same biological activity as the unmodified form, in vivo and/or in vitro.

The term "angiogenesis" refers generally to the formation and differentiation of blood vessels.

The term "antibody" as used herein describes antibodies, fragments of antibodies (such as, but not limited, to Fab, Fab', Fab₂', and Fv fragments), and chimeric, humanized, veneered, resurfaced, or CDR-grafted antibodies capable of binding antigens of a similar nature as the parent antibody molecule from which they are derived.

The term "chimeric protein" or "fusion protein" refers to a hybrid protein or peptide comprising part or all of a Scarface 1 protein or analog thereof linked to a heterologous protein. The heterologous protein can be fused to the N-terminus or the C-terminus of Scarface 1. A preferred chimeric protein comprises the fibrinogen-like domain of a Scarface 1 protein fused to the constant region of an antibody.

The terms "complementary" or "complementarity" as used herein refer to the capacity of purine and pyrimidine nucleotides to associate through hydrogen bonding to form double stranded nucleic acid molecules. The following base pairs are related by complementarity: guanine and cytosine; adenine and thymine; and adenine and uracil. As used herein, "complementary" means that the aforementioned relationship applies to substantially all base pairs comprising two single-stranded nucleic acid molecules over the entire length of said molecules. "Partially complementary" refers to the aforementioned relationship in which one of two single-stranded nucleic acid molecules is shorter in length than the other such that a portion of one of the molecules remains single-stranded.

The term "conservative substitution" or "conservative amino acid substitution" refers to a replacement of one or more amino acid residue(s) in a parent protein as stipulated by Table 1.

The term "fibrinogen-like domain" refers to a conserved region of amino acids typically found at the carboxy end of a protein molecule that contains such a domain, said domain being related structurally and/or by sequence to the homologous region of a fibrinogen protein molecule. For example, the fibrinogen-like domain of Scarface 1 resides between the position approximating residues 275 through 493 of SEQ ID NO:2. The term is intended to include domains that are at least 65% identical with residues 275 through 493 of SEQ ID NO:2; alternatively, between 70% to 100% identical; preferably, greater than 85% identical; more preferably, at least 90% identical; and most preferably, greater than 95% identical. The fibrinogen-like domain is thought to be important for the biological activity of Scarface 1 and related molecules, for example, as the receptor binding site.

The term "fragment thereof" refers to a fragment, piece, or sub-region of a nucleic acid or protein molecule whose sequence is disclosed herein, such that the fragment comprises 5 or more amino acids, or 10 or more nucleotides that are contiguous in the parent protein or nucleic acid molecule. "Fragment thereof" may or may not retain biological activity. A fragment of a protein disclosed herein could be used as an antigen to raise a specific antibody against the parent protein molecule. With reference to a nucleic acid molecule, "fragment thereof" refers to 10 or more contiguous nucleotides, derived from a parent nucleic acid. The term also encompasses the complementary sequence. For example if the fragment entails the sequence 5'-AGCTAG-3', then "fragment thereof" would also include the complementary sequence, 3'-TCGATC-5'.

The term "functional fragment" or "functionally equivalent fragment", as used herein, refers to a region, or fragment of a full length protein, or sequence of amino acids that, for example, comprises an active site, or any other motif, relating to biological function. Functional fragments are capable of providing a substantially similar biological activity as a protein disclosed herein, *in vivo* or *in vitro,* viz. the capacity to modulate angiogenesis, or the ability to promote phosphorylation of the Tie2 receptor on, for example, epithelial cells. Functional fragments may be produced by cloning technology, or as the natural products of alternative splicing mechanisms.

The term "functionally related" is used herein to describe peptides that are related to the Scarface 1 peptides of the present invention, said functionally related peptides constituting modifications of said Scarface 1 peptides in which conservative or other suitable amino acid changes are present, or are introduced by natural mutation or by recombinant DNA techniques. Functionally related peptides retain the biological activity of Scarface 1 peptides, such as the ability to modulate angiogenesis, or the ability to stimulate phosphorylation of Tie2 *(See e.g.* D. Dumont et al. *Oncogene* 7, 1471-1480, 1992).

The term "structurally and functionally related" is used herein to describe peptides or proteins that are related structurally and functionally to Scarface 1. Said proteins, generally constitute modifications of SEQ ID NO:2 in which conservative or other suitable amino acid changes are introduced by natural mutation or by recombinant DNA techniques, or by other means, or by deletion of one to ten contiguous amino acid residues of SEQ ID NO:2. Generally, structurally and functionally related proteins are greater than 50% identical with SEQ ID NO:2, alternatively greater than 65% identical, preferably between 70% to 100% identical with SEQ ID NO:2; still more preferably between 85% to 95% identical; more preferably still, at least 90% identical; most preferably at least 95% identical; still most preferably between 98% to 100% identical to SEQ ID NO:2. The overall percent identity between nucleic acid molecules or protein molecules is determined by any suitable comparison algorithm, well known to the skilled artisan. The overall percent identity is determined by the number of residues that are identical between optimally aligned nucleic acids or proteins, divided by the total length (including gaps if present) of the shortest sequence of the pair or group being compared.

Percent identity can also refer to, or be expressed as, a local comparison of regions, motifs, or blocks within molecules. Local identities between molecules may be greater than 80% to 90%, while surrounding regions may be less conserved, for example, less than 50% identical. Such variability in sequence conservation along the length of molecules can produce a low percentage identity overall, but a high percentage identity within locally defined regions. Examples of locally conserved regions might include binding sites, or catalytic sites. The invention is therefore intended to include analogs of Scarface 1 that are at least 40% identical with the fibrinogen binding region comprising residues from about 275 through 493 of SEQ ID NO:2.

Nucleic acid or protein sequences are optimally aligned so as to achieve the greatest degree of similarity, allowing for gaps, using any suitable algorithm, for example, a dynamic programming algorithm (See e.g. Smith and Waterman, J. Mol. Biol. 147, 195 (1985), BLASTA (Altschul et al. J. Mol. Biol. 215, 403 (1990); or FASTA (Lipman & Pearson, Science, 227, 1435 (1985), herein incorporated by reference. Such alignments are carried out with the appropriate algorithmic parameters set to maximize the alignment score obtained for a pair of sequences being compared. For example, using a BLAST algorithm, the Hsp parameter can be set at from 10 to 11 for comparing nucleic acids and at 3 when comparing amino acid sequences. Other parameters that can be varied include the expectation value parameter, most typically set to 10. The skilled artisan is aware of the values most appropriate so as to maximize the alignment between any two or more sequences. Said structurally and functionally related proteins retain the biological activity of Scarface 1, such as the ability to modulate angiogenesis, or the ability to stimulate phosphorylation of Tie2, or to inhibit solid tumor growth in vivo, or the ability to suppress the growth of hematopoietic progenitor cells *(See e.g.* D. Dumont et al. *Oncogene* 7, 1471-1480, 1992).

Thus, the present invention comprises Scarface 1, as defined by SEQ ID NO:2, as well as functionally equivalent variants comprising naturally occurring allelic variations, as well as proteins or peptides comprising substitutions, deletions or insertional mutants of SEQ ID NO:2, which bind TIE receptor and act as agonists or antagonists thereof. Such variants include those in which amino acid residues are substituted for residues within SEQ ID NO:2 resulting in a silent change. For example, one or more amino acid residues within the sequence can be substituted by another amino acid(s) of a similar polarity which acts as a functional equivalent, resulting in a silent alteration. Substitutes for an amino acid within the sequence may be selected from other members of the class to which the amino acid belongs. For example, the class of nonpolar (hydrophobic) amino acids include alanine, leucine, isoleucine, valine, proline, phenylalanine, tryptophan and methionine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid. Also included within the scope of the invention are proteins or fragments or derivatives thereof which exhibit the same or similar biological activity as the Scarface 1 described herein, and derivatives which are differentially modified during or after translation, e.g., by glycosylation, proteolytic cleavage, linkage to an antibody molecule or other cellular ligand, etc.

The term "host cell" refers to any eucaryotic or procaryotic cell that is suitable for propagating and/or expressing a cloned gene contained on a vector that is introduced into said host cell by, for example, transformation or transfection, or the like.

Specific examples of Scarface 1 genes and/or proteins are referred to herein as "Scarface 1" (SEQ ID NO:1/SEQ ID NO:2). Scarface 1 molecules are related to angiopoietin 1, which mediates angiogenesis during embryogenic and later development.

The term "homolog" or "homologous" describes the relationship between different nucleic acid molecules, or amino acid sequences, in which said sequences or molecules are related by partial identity or chemical/physical similarity at one or more blocks or regions within said molecules or sequences.

The term "hybridization" as used herein refers to a process in which a single-stranded nucleic acid molecule joins with a complementary strand through nucleotide base pairing. "Selective hybridization" refers to hybridization under conditions of high stringency. The degree of hybridization depends upon, for example, the degree of homology, the stringency of hybridization, and the length of hybridizing strands.

The term "isolated nucleic acid compound" refers to any RNA or DNA sequence, however constructed or synthesized, which is locationally distinct from its natural location.

The term "modulate" or "modulating" refers to stimulating or inhibiting the object thereof, for example, stimulating or inhibiting angiogenesis.

The symbol "N" in a nucleic acid sequence refers to adenine ("A"), guanine ("G"), cytosine ("C"), thymine (T"), or uracil ("U").

The term "nucleic acid probe" or "probe" as used herein is a labeled nucleic acid compound which hybridizes with another nucleic acid compound. "Nucleic acid probe" means a single stranded nucleic acid sequence that will combine with a complementary or partially complementary single stranded target nucleic acid sequence to form a double-stranded molecule. A nucleic acid probe may be an oligonucleotide or a nucleotide polymer. A probe will usually contain a detectable moiety which may be attached to the end(s) of the probe or be internal to the sequence of the probe.

The term "orthologue" or "orthologous" refers to two or more genes or proteins from different organisms that exhibit sequence homology.

The term "paralogue" or "paralogous" refers to two or more genes or proteins within a single organism that exhibit sequence homology.

The term "peptide" or "protein" are used interchangeably to mean a biopolymer comprising a linear array of two or more peptide bonded amino acids.

The term "plasmid" refers to an extrachromosomal genetic element. The plasmids disclosed herein are commercially available, publicly available on an unrestricted basis, or can be constructed from readily available plasmids in accordance with published procedures.

A "primer" is a nucleic acid fragment which functions as an initiating substrate for enzymatic or synthetic elongation of, for example, a nucleic acid molecule.

The term "promoter" refers to a nucleic acid sequence that directs transcription, for example, of DNA to RNA. An inducible promoter is one that is regulatable by environmental signals, such as carbon source, heat, or metal ions, for example. A constitutive promoter generally operates at a constant level and is not regulatable.

The term "recombinant DNA cloning vector" as used herein refers to any autonomously replicating agent, including, but not limited to, plasmids and phages, comprising a DNA molecule to which one or more additional DNA segments can or have been incorporated.

The term "recombinant DNA expression vector" or "expression vector" as used herein refers to any recombinant DNA cloning vector, for example a plasmid or phage, in which a promoter and other regulatory elements are present thereby enabling transcription of an inserted DNA, which may encode a protein.

The term "Scarface 1 activity" or "Scarface 1-like activity" refers to one or more biological activities exhibited by Scarface 1, for example, the ability to modulate angiogenesis, in vivo or any suitable in vitro proxy assay, or the ability to stimulate phosphorylation of Tie 2, or any other biological effect or activity of Scarface 1 or a functional region of Scarface 1, for example, the fibrinogen-like domain.

The term "stem cell" refers generally to an unspecialized cell type that gives rise to differentiated cells. For example, hematopoietic stem cells in bone marrow give rise to cells of the blood, e.g. B-cells and red blood cells.

The term "stringency" refers to hybridization conditions. High stringency conditions disfavor non-homologous base pairing. Low stringency conditions have the opposite effect. Stringency may be altered, for example, by temperature and salt concentration.

The term "low stringency" refers to conditions that comprise, for example, a temperature of about 37° C or less, a formamide concentration of less than about 50%, and a moderate to low salt (SSC) concentration; or, alternatively, a temperature of about 50° C or less, and a moderate to high salt (SSPE) concentration, for example 1M NaCl.

The term "high stringency" refers to conditions that comprise, for example, a temperature of about 42° C or less, a formamide concentration of less than about 20%, and a low salt (SSC) concentration; or, alternatively, a temperature of about 65° C, or less, and a low salt (SSPE) concentration. For example, high stringency conditions comprise hybridization in 0.5 M NaHPO₄, 7% sodium dodecyl sulfate (SDS), 1 mM EDTA at 65°C (Ausubel, F.M. *et al.* Current Protocols in Molecular Biology, Vol. I, 1989; Green Inc. New York, at 2.10.3).

The term "SSC" comprises a hybridization and wash solution. A stock 20X SSC solution contains 3M sodium chloride, 0.3M sodium citrate, pH 7.0.

The term "SSPE" comprises a hybridization and wash solution. A 1X SSPE solution contains 180 mM NaCl, 9mM Na₂HPO₄, 0.9 mM NaH₂PO₄ and 1 mM EDTA, pH 7.4.

The term "substantially pure," used in reference to a peptide or protein, means separation from other cellular and non-cellular molecules, including other protein molecules. A substantially pure preparation would be about at least 85% pure; preferably about at least 95% pure. A "substantially pure" protein can be prepared by a variety of techniques, well known to the skilled artisan, including, for example, the IMAC protein purification method.

The term "vector" as used herein refers to a nucleic acid compound used for introducing exogenous or endogenous DNA into host cells. A vector comprises a nucleotide sequence which may encode one or more protein molecules. Plasmids, cosmids, viruses, and bacteriophages, in the natural state or which have undergone recombinant engineering, are examples of commonly used vectors.

The various restriction enzymes disclosed and described herein are commercially available and the manner of use of said enzymes including reaction conditions, cofactors, and other requirements for activity are well known to one of ordinary skill in the art. Reaction conditions for particular enzymes were carried out according to the manufacturer's recommendation.

The Scarface 1 genes encode proteins that are related to mammalian angiopoietin 1 and to the *Drosophila* "Scabrous" protein. Species of Scarface 1 DNA sequences are disclosed herein by SEQ ID NO:1 (Scarface 1). Those skilled in the art will recognize that owing to the degeneracy of the genetic code, numerous "silent" substitutions of nucleotide base pairs could be introduced into the sequences identified herein without altering the identity of the encoded amino acid(s) or protein or peptide product. All such substitutions are intended to be within the scope of the invention.

Specific examples of a Scarface 1 cDNA and protein are identified herein as SEQ ID NO:1 and SEQ ID NO:2, respectively. Scarface 1 (SEQ ID NO:2) comprises multiple functional domains including a fibrinogen-like domain from approximately about residue 275 through 493, and a coiled-coil domain at the amino terminal end from approximately residues 1 through 274.

Also contemplated by the present invention are peptides that are functionally related to Scarface 1. For example, peptides that are functionally related to SEQ ID NO:2 may be produced by conservative amino acid substitutions, replacements, deletions, or insertions, at one or more amino acid positions within Scarface 1, in accordance with the Table presented herein.

Modifications of Scarface 1 peptides made in accordance with the Table are generally expected to retain the biological activity of the parent molecule based on the art recognized substitutability of certain amino acids (*See e.g.* M. Dayhoff, In Atlas of Protein Sequence and Structure, Vol. 5, Supp. 3, pgs 345-352, 1978). Functionality is easily tested in an assay that measures phosphorylation of the Tie2 receptor, for example.

| ORIGINAL RESIDUE | EXEMPLARY SUBSTITUTIONS |
|---|---|
| ALA | SER, THR |
| ARG | LYS |
| ASN | HIS, SER |
| ASP | GLU, ASN |
| CYS | SER |
| GLN | ASN, HIS |
| GLU | ASP, GLU |
| GLY | ALA, SER |
| HIS | ASN, GLN |
| ILE | LEU, VAL, THR |
| LEU | ILE, VAL |
| LYS | ARG, GLN, GLU, THR |
| MET | LEU, ILE, VAL |
| PHE | LEU, TYR |
| SER | THR, ALA, ASN |
| THR | SER, ALA |
| TRP | ARG, SER |
| TYR | PHE |
| VAL | ILE, LEU, ALA |
| PRO | ALA |

### Fragments of Scarface 1 proteins

One embodiment of the instant invention provides fragments of the peptides disclosed that may or may not be biologically active. Such fragments are useful, for example, as an antigen for producing an antibody to said proteins.

Fragments of the proteins disclosed herein may be generated by any number of suitable techniques, including chemical synthesis of any portion of SEQ ID NO:2, proteolytic digestion of said peptides, or most preferably, by recombinant DNA mutagenesis techniques, well known to the skilled artisan. *See. e.g.* K. Struhl, "Reverse biochemistry: Methods and applications for synthesizing yeast proteins *in vitro," Meth. Enzymol.* 194, 520-535. For example, in a preferred method, a nested set of deletion mutations are introduced into a gene or gene fragment (e.g. SEQ ID NO:1) encoding an Scarface 1 peptide such that varying amounts of the peptide coding region are deleted, either from the amino terminal end, or from the carboxyl end of the protein molecule. This method can also be used to create internal fragments of the intact protein in which both the carboxyl and amino terminal ends are removed. Several appropriate nucleases can be used to create such deletions, for example *Bal*31*,* or in the case of a single stranded nucleic acid molecule, mung bean nuclease. For simplicity, it is preferred that the Scarface 1 gene be cloned into a single-stranded cloning vector, such as bacteriophage M13, or equivalent. If desired, the resulting deletion fragments can be subcloned into any suitable vector for propagation and expression in any suitable host cell.

Functional fragments of the proteins of this invention may be produced as described above, preferably using cloning techniques to engineer smaller versions of the intact gene, lacking sequence from the 5' end, the 3' end, from both ends, or from an internal site. Smaller fragments of the genes of this invention may be used to produce the encoded peptides, which may be tested for biological activity using any suitable assay, for example, the ability of a protein fragment to stimulate phosphorylation of Tie2.

### Gene Isolation Procedures

Those skilled in the art will recognize that the Scarface 1 genes could be obtained by a plurality of recombinant DNA techniques including, for example, hybridization, polymerase chain reaction (PCR) amplification, or *de novo* DNA synthesis. (*See e.g.,* T. Maniatis *et al.* Molecular Cloning: A Laboratory Manual, 2d Ed. Chap. 18 (1989)).

Methods for constructing cDNA libraries in a suitable vector such as a plasmid or phage for propagation in procaryotic or eucaryotic cells are well known to those skilled in the art. [*See e.g.* Maniatis *et al. Supra*]*.* Suitable cloning vectors are well known and are widely available.

The Scarface 1 genes, or fragments thereof, can be isolated from any tissue in which said genes are expressed. For example, Northern analysis reveals that Scarface 1 is expressed strongly in muscle, bone marrow, spleen, and lymph node, and at lower levels in thymus and fetal liver.

In one method for gene isolation, mRNA is isolated from a suitable tissue, and first strand cDNA synthesis is carried out. Second round DNA synthesis can be carried out for the production of the second strand. If desired, the double-stranded cDNA can be cloned into any suitable vector, for example, a plasmid, thereby forming a cDNA library. Oligonucleotide primers targeted to any suitable region of the sequences disclosed herein can be used for PCR amplification of Scarface 1 genes. *See e.g.* PCR Protocols: A Guide to Method and Application, Ed. M. Innis et al., Academic Press (1990). The PCR amplification comprises template DNA, suitable enzymes, primers, and buffers, and is conveniently carried out in a DNA Thermal Cycler (Perkin Elmer Cetus, Norwalk, CT). A positive result is determined by detecting an appropriately-sized DNA fragment following agarose gel electrophoresis.

### Protein Production Methods

One embodiment of the present invention relates to substantially purified proteins encoded by Scarface 1 genes.

Skilled artisans will recognize that the proteins of the present invention can be synthesized by a number of different methods, such as chemical methods well known in the art, including solid phase peptide synthesis or recombinant methods. Both methods are described in U.S. Patent 4,617,189, incorporated herein by reference.

The principles of solid phase chemical synthesis of polypeptides are well known in the art and may be found in general texts in the area. *See, e.g.*, H. Dugas and C. Penney, Bioorganic Chemistry (1981) Springer-Verlag, New York, 54-92. For example, peptides may be synthesized by solid-phase methodology utilizing an Applied Biosystems 430A peptide synthesizer (Applied Biosystems, Foster City, CA) and synthesis cycles supplied by Applied Biosystems.

The proteins of the present invention can also be produced by recombinant DNA methods using a cloned Scarface 1 gene as template. Recombinant methods are preferred if a high yield is desired. Expression of an Scarface 1 gene can be carried out in a variety of suitable host cells, well known to those skilled in the art. For example, SEQ ID NO:1 is introduced into a host cell by any suitable means, well known to those skilled in the art. While chromosomal integration of the cloned gene is within the scope of the present invention, it is preferred that the gene be cloned into a suitable extra-chromosomally maintained expression vector so that the coding region of the Scarface 1 gene is operably-linked to a constitutive or inducible promoter.

The basic steps in the recombinant production of an Scarface 1 protein are:
a) constructing a natural, synthetic or semi-synthetic DNA encoding an Scarface 1 protein;
b) integrating said DNA into an expression vector in a manner suitable for expressing the Scarface 1 protein, either alone or as a fusion protein;
c) transforming or otherwise introducing said vector into an appropriate eucaryotic or prokaryotic host cell forming a recombinant host cell,
d) culturing said recombinant host cell in a manner to express the Scarface 1 protein; and
e) recovering and substantially purifying the Scarface 1 protein by any suitable means, well known to those skilled in the art.
In addition, the invention further contemplates compositions comprising the receptor binding domain of Scarface 1. For example, Scarface 1 consists of a "coiled coil" domain (beginning at the amino terminal end and extending to about position 274 of SEQ ID NO:2) and a fibrinogen-like domain (beginning at about position 275 and extending through the C terminal end). Multimerization of the coiled-coil domains during production of the protein hampers purification. The fibrinogen-like domain is likely to comprise the TIE-2 receptor binding domain. The monomeric forms of the fibrinogen-like domain do not, however, appear to bind the receptor. Methods of production of "clustered ligands and ligandbodies are described in Davis, et al. Science 266:816-819 (1994). Based on these findings the invention further relates to "ligandbodies," or Scarface 1 fusion proteins, which comprise the fibrinogen-like domain of Scarface 1 coupled to the Fc domain of IgG, for example. These fusion proteins form dimers that bind the TIE-2 receptor. Accordingly, the present invention contemplates the production of Scarface 1 fusion proteins which may be used as targeting agents, in diagnostics or in therapeutic applications, such as targeting agents for tumors and/or associated vasculature wherein a TIE antagonist is indicated.

The invention herein further provides for the development of the Scarface 1 ligand, a fragment or derivative thereof, or another molecule which is a receptor agonist or antagonist, as a therapeutic for the treatment of patients suffering from disorders involving cells, tissues or organs which express the TIE receptor. Such molecules may be used in a method of treatment of the human or animal body, or in a method of diagnosis.

Because TIE receptor has been identified in association with endothelial cells, applicants expect that Scarface 1 may be useful for the induction of vascularization in diseases or disorders where such vascularization is indicated. Such diseases or disorders would include wound healing, ischaemia and diabetes. The ligands may be tested in animal models and used therapeutically as described for other agents, such as vascular endothelial growth factor (VEGF), another endothelial cell- specific factor that is angiogenic.

For example, various in vivo and/or in vitro studies are useful to demonstrate the effect of an angiogenic factor in enhancing blood flow to ischemic myocardium, enhancing wound healing, and in other therapeutic settings wherein neoangiogenesis is desired. See Ferrara, et al. U.S. Pat. No. 5,332,671; European Patent Application 0 550 296 A2; Banai, et al. Circulation 89, 2183-2189 (1994); Unger, et al. Am. J. Physiol. 266, H1588-H1595 (1994); Lazarous, et al. Circulation 91, 145-153 (1995), all of which are herein incorporated by reference.

According to the invention, Scarface 1 may be used alone or in combination with one or more additional pharmaceutically active compounds such as, for example, VEGF or basic fibroblast growth factor (bFGF), as well as cytokines, neurotrophins, etc.

### Expressing Recombinant Scarface 1 Protein in Procaryotic and Eucaryotic Host Cells

Procaryotes may be employed in the production of recombinant Scarface 1 protein. For example, the *Escherichia coli* K12 strain 294 (ATCC No. 31846) is particularly useful for the prokaryotic expression of foreign proteins. Other strains of *E. coli,* bacilli such as *Bacillus subtilis,* enterobacteriaceae such as *Salmonella typhimurium* or *Serratia marcescens,* various *Pseudomonas* species and other bacteria, such as *Streptomyces,* may also be employed as host cells in the cloning and expression of the recombinant proteins of this invention.

Promoter sequences suitable for driving the expression of genes in procaryotes include b -lactamase [*e.g.* vector pGX2907, ATCC 39344, contains a replicon and b -lactamase gene], lactose systems [Chang et al., *Nature* (London), 275:615 (1978); Goeddel et al., *Nature* (London), 281:544 (1979)], alkaline phosphatase, and the tryptophan (trp) promoter system [vector pATH1 (ATCC 37695)], which is designed to facilitate expression of an open reading frame as a trpE fusion protein under the control of the trp promoter. Hybrid promoters such as the tac promoter (isolatable from plasmid pDR540, ATCC-37282) are also suitable. Still other bacterial promoters, whose nucleotide sequences are generally known, may be ligated to DNA encoding the protein of the instant invention, using linkers or adapters to supply any required restriction sites. Promoters for use in bacterial systems also will contain a Shine-Dalgarno sequence operably-linked to the DNA encoding the desired polypeptides. These examples are illustrative rather than limiting.

The proteins of this invention may be synthesized either by direct expression or as a fusion protein comprising the protein of interest as a translational fusion with another protein or peptide which may be removable by enzymatic or chemical cleavage. It is often observed in the production of certain peptides in recombinant systems that expression as a fusion protein prolongs the lifespan, increases the yield of the desired peptide, or provides a convenient means of purifying the protein. This is particularly relevant when expressing mammalian proteins in procaryotic hosts. A variety of peptidases (e.g. enterokinase and thrombin) which cleave a polypeptide at specific sites or digest the peptides from the amino or carboxy termini (*e.g.* diaminopeptidase) of the peptide chain are known. Furthermore, particular chemicals (*e.g.* cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. *See e.g.,* P. Carter, "Site Specific Proteolysis of Fusion Proteins", Chapter 13, in Protein Purification: From Molecular Mechanisms to Large Scale Processes, American Chemical Society, Washington, D.C. (1990).

One useful Scarface 1 fusion protein is a GST fusion protein in which a Scarface 1 protein of the invention is fused to the C-terminus of GST sequences. Such fusion proteins are useful, for example, for purification of a recombinant polypeptide of the invention.

In another embodiment of the invention, the fusion partner comprises an immunoglobulin peptide or protein in which part or all of a Scarface 1 protein or analog, for example, the fibrinogen-like domain, is fused to the constant region of an IgG molecule. Such fusion proteins can facilitate purification and may be useful in pharmaceutical compositions or in screens for compounds that modulate the interaction of Scarface 1 with its receptor.

In addition to procaryotes, a variety of amphibian expression systems such as frog oocytes, and mammalian cell systems can be used. The choice of a particular host cell depends to some extent on the particular expression vector used. Exemplary mammalian host cells suitable for use in the present invention include HepG-2 (ATCC HB 8065), CV-1 (ATCC CCL 70), LC-MK₂ (ATCC CCL 7.1), 3T3 (ATCC CCL 92), CHO-K1 (ATCC CCL 61), HeLa (ATCC CCL 2), RPMI8226 (ATCC CCL 155), H4IIEC3 (ATCC CCL 1600), C127I (ATCC CCL 1616), HS-Sultan (ATCC CCL 1884), and BHK-21 (ATCC CCL 10), for example.

A wide variety of vectors are suitable for transforming mammalian host cells. For example, the pSV2-type vectors comprise segments of the simian virus 40 (SV40) genome required for transcription and polyadenylation. A large number of plasmid pSV2-type vectors have been constructed, such as pSV2-gpt, pSV2-neo, pSV2-dhfr, pSV2-hyg, and pSV2-b-globin, in which the SV40 promoter drives transcription of an inserted gene. These vectors are widely available from sources such as the American Type Culture Collection (ATCC), 12301 Parklawn Drive, Rockville, Maryland, 20852, or the Northern Regional Research Laboratory (NRRL), 1815 N. University Street, Peoria, Illinois, 61604.

Promoters suitable for expression in mammalian cells include the SV40 late promoter, promoters from eukaryotic genes, such as, for example, the estrogen-inducible chicken ovalbumin gene, the interferon genes, the glucocorticoid-inducible tyrosine aminotransferase gene, the thymidine kinase gene promoter, and the promoters of the major early and late adenovirus genes.

Plasmid pRSVcat (ATCC 37152) comprises portions of a long terminal repeat of the Rous Sarcoma virus, a virus known to infect chickens and other host cells. This long terminal repeat contains a promoter which is suitable for use in the vectors of this invention. H. Gorman *et al., Proc. Nat. Acad. Sci. (USA)*, 79, 6777 (1982). The plasmid pMSVi (NRRL B-15929) comprises the long terminal repeats of the Murine Sarcoma virus, a virus known to infect mouse and other host cells. The mouse metallothionein promoter has also been well characterized for use in eukaryotic host cells and is suitable for use in the present invention. This promoter is present in the plasmid pdBPV-MMTneo (ATCC 37224) which can serve as the starting material for the construction of other plasmids of the present invention.

Transfection of mammalian cells with vectors can be performed by a plurality of well known processes including, but not limited to, protoplast fusion, calcium phosphate co-precipitation, electroporation and the like. *See, e.g.,* Maniatis *et al., supra.*

Some viruses also make appropriate vectors. Examples include the adenoviruses, the adeno-associated viruses, the vaccinia virus, the herpes viruses, the baculoviruses, and the rous sarcoma virus, as described in U.S. Patent 4,775,624, incorporated herein by reference.

Eucaryotic microorganisms such as yeast and other fungi are also suitable host cells. The yeast *Saccharomyces cerevisiae* is the preferred eucaryotic microorganism. Other yeasts such as *Kluyveromyces lactis* and *Pichia pastoris* are also suitable. For expression in *Saccharomyces,* the plasmid YRp7 (ATCC-40053), for example, may be used. *See, e.g.,* L. Stinchcomb *et al., Nature,* 282, 39 (1979); J. Kingsman *et al., Gene,* 7, 181 (1979); S. Tschemper *et al., Gene,* 10, 157 (1980). Plasmid YRp7 contains the TRP1 gene which provides a selectable marker for use in a trpl auxotrophic mutant.

### Purification of Recombinantly-Produced Scarface 1 Proteins

An expression vector carrying a cloned Scarface 1 gene or fragment thereof (e.g. SEQ ID NO:1) is transformed or transfected into a suitable host cell using standard methods. Cells that contain the vector are propagated under conditions suitable for expression of a recombinant Scarface 1 protein. For Example, if the recombinant gene or fragment thereof has been placed under the control of an inducible promoter, suitable growth conditions would incorporate the appropriate inducer. The recombinantly-produced protein may be purified from cellular extracts of transformed cells by any suitable means.

In a preferred process for protein purification, an Scarface 1 gene or fragment thereof is modified at the 5' end to incorporate several histidine codons. This modification produces an "histidine tag" at the amino terminus of the encoded protein, that enables a single-step protein purification method [i.e. "immobilized metal ion affinity chromatography" (IMAC)], essentially as described in U.S. Patent 4,569,794, which hereby is incorporated by reference. The IMAC method enables rapid isolation of substantially pure recombinant Scarface 1 protein starting from a crude extract of cells that express a modified recombinant protein, as described above.

### Production of Antibodies

The proteins of this invention and fragments thereof may be used in the production of antibodies that selectively bind Scarface 1. The instant invention also encompasses single-chain polypeptide binding molecules.

The production of antibodies, both monoclonal and polyclonal, in animals, especially mice, is well known in the art. *See, e.g.*, C. Milstein, Handbook of Experimental Immunology, (Blackwell Scientific Pub., 1986); J. Goding, Monoclonal Antibodies: Principles and Practice, (Academic Press, 1983). For the production of monoclonal antibodies the basic process begins with injecting a mouse, or other suitable animal, with an immunogen. The mouse is subsequently sacrificed and cells taken from its spleen are fused with myeloma cells, resulting in a hybridoma that reproduces *in vitro.* The population of hybridomas is screened to isolate individual clones, each of which secretes a single antibody species, specific for the immunogen. Each antibody obtained in this way is the clonal product of a single B cell.

Chimeric antibodies are described in U.S. Patent No. 4,816,567, the entire contents of which is herein incorporated by reference. This reference discloses methods and vectors for the preparation of chimeric antibodies. An alternative approach is provided in U.S. Patent No. 4,816,397, the entire contents of which is herein incorporated by reference. This patent teaches co-expression of the heavy and light chains of an antibody in the same host cell.

The approach of U.S. Patent 4,816,397 has been further refined in European Patent Publication No. 0 239 400. The teachings of this European patent publication are a preferred format for genetic engineering of monoclonal antibodies. In this technology the complementarity determining regions (CDRs) of a human antibody are replaced with the CDRs of a murine monoclonal antibody, thereby converting the specificity of the human antibody to the specificity of a murine antibody.

Single chain antibodies and libraries thereof are yet another variety of genetically engineered antibody technology that is well known in the art. *(See, e.g.* R.E. Bird, *et al., Science* 242:423-426 (1988); PCT Publication Nos. WO 88/01649, WO 90/18430, and WO 91/10737. Single chain antibody technology involves covalently joining the binding regions of heavy and light chains to generate a single polypeptide chain. The binding specificity of the intact antibody molecule is thereby reproduced on a single polypeptide chain.

The antibodies contemplated by this invention are useful in diagnostics, therapeutics or in diagnostic/therapeutic combinations.

The Scarface 1 proteins or analogs of this invention or suitable fragments thereof can be used to generate polyclonal or monoclonal antibodies, and various interspecies hybrids, or humanized antibodies, or antibody fragments, or single-chain antibodies. The techniques for producing antibodies are well known to skilled artisans. (*See e.g.* A.M. Campbell, Monoclonal Antibody Technology: Laboratory Techniques in Biochemsitry and Molecular Biology, Elsevier Science Publishers, Amsterdam (1984); Kohler and Milstein, *Nature* 256, 495-497 (1975); Monoclonal Antibodies: Principles & Applications Ed. J.R.Birch & E.S. Lennox, Wiley-Liss, 1995.

A protein used as an immunogen may be modified or administered in an adjuvant, by subcutaneous or intraperitoneal injection into, for example, a mouse or a rabbit. For the production of monoclonal antibodies, spleen cells from immunized animals are removed, fused with myeloma cells, such as SP2/0-Ag18 cells, and allowed to become monoclonal antibody producing hybridoma cells in the manner known to the skilled artisan. Hybridomas that secrete a desired antibody molecule can be screened by a variety of well known methods, for example ELISA assay, western blot analysis, or radioimmunoassay (Lutz *et al. Exp. Cell Res.* 175, 109-124 (1988); Monoclonal Antibodies: Principles & Applications Ed. J.R.Birch & E.S. Lennox, Wiley-Liss, 1995).

For some applications labeled antibodies are desirable. Procedures for labeling antibody molecules are widely known, including for example, the use of radioisotopes, affinity labels, such as biotin or avidin, enzymatic labels, for example horseradish peroxidase, and fluorescent labels, such as FITC or rhodamine (*See e.g.* Enzyme-Mediated Immunoassay, Ed. T. Ngo, H. Lenhoff, Plenum Press 1985; Principles of Immunology and Immunodiagnostics, R.M. Aloisi, Lea & Febiger, 1988).

Labeled antibodies are useful for a variety of diagnostic applications. In one embodiment the present invention relates to the use of labeled antibodies to detect the presence of Scarface 1 proteins. Alternatively, the antibodies could be used in a screen to identify potential modulators of Scarface 1. For example, in a competitive displacement assay, the antibody or compound to be tested is labeled by any suitable method. Competitive displacement of an antibody from an antibody-antigen complex by a test compound such that a test compound-antigen complex is formed provides a method for identifying compounds that bind Scarface 1.

Other embodiments of the present invention comprise isolated nucleic acid sequences that encode the proteins of the invention, for example, SEQ ID NO:2. Also contemplated are related nucleic acids that hybridize to SEQ ID NO:1 under high stringency conditions. Such sequences may come, for example, from paralogous or orthologous genes.

The Scarface 1 genes of the invention (e.g. SEQ ID NO:1) and related nucleic acid molecules may be produced by chemical synthetic methods. The synthesis of nucleic acids is well known in the art. *See, e.g.,* E.L. Brown, R. Belagaje, M.J. Ryan, and H.G. Khorana, *Methods in* *Enzymology,* 68:109-151 (1979). Nucleic acids of this invention, including those disclosed herein, could be generated using a conventional DNA synthesizing apparatus, such as the Applied Biosystems Model 380A or 380B DNA synthesizers (Applied Biosystems, Inc., 850 Lincoln Center Drive, Foster City, CA 94404) using phosphoramidite chemistry, thereafter ligating the fragments so as to reconstitute the entire gene. Alternatively, phosphotriester chemistry may be employed to synthesize the nucleic acids of this invention. (*See, e.g.,* M.J. Gait, ed., Oligonucleotide Synthesis, A Practical Approach, (1984)).

In an alternative methodology, namely PCR, the DNA sequences disclosed and described herein, can be produced from a plurality of starting materials. For example, starting with a cDNA preparation (e.g. cDNA library) derived from a tissue that expresses an Scarface 1 gene, suitable oligonucleotide primers complementary to, for example, SEQ ID NO:1, or to a sub-region therein, are prepared as described in U.S. Patent No. 4,889,818, hereby incorporated by reference. Other suitable protocols for the PCR are disclosed in PCR Protocols: A Guide to Method and Applications, Ed. Michael A. Innis et al., Academic Press, Inc. (1990). Using PCR, any region of the Scarface 1 gene(s) can be targeted for amplification such that full or partial length gene sequences may be produced.

The ribonucleic acids of the present invention may be prepared using polynucleotide synthetic methods discussed *supra,* or they may be prepared enzymatically, for example, using RNA polymerase to transcribe a Scarface 1 DNA template.

The most preferred systems for preparing the ribonucleic acids of the present invention employ the RNA polymerase from the bacteriophage T7 or the bacteriophage SP6. These RNA polymerases are highly specific, requiring the insertion of bacteriophage-specific sequences at the 5' end of the template to be transcribed. See, Maniatis *et al., supra.*

This invention also provides nucleic acids, RNA or DNA, that are complementary to Scarface 1 genes or fragments thereof, for example, SEQ ID NO:1.

### Nucleic Acid Probes

The present invention also provides probes and primers useful, for example, in hybridization screens of genomic, subgenomic, or cDNA libraries, as well as hybridization against nucleic acids derived from cell lines or tissues. Such hybridization screens are useful as methods to identify and isolate full length, or identical, homologous or functionally related sequences from human or other organisms.

In one embodiment the present invention relates to the use of a nucleic acid disclosed herein as a probe to identify and isolate full-length genes comprising said nucleic acids. A nucleic acid compound comprising SEQ ID NO:1, or a complementary sequence thereof, or a fragment thereof, which is at least 18 base pairs in length, and which will selectively hybridize to DNA or mRNA encoding Scarface 1 protein or fragment thereof, or a functionally related protein, is provided. Preferably, the 18 or more base pair compound is DNA. *See e.g.* B. Wallace and G. Miyada, "Oligonucleotide Probes for the Screening of Recombinant DNA Libraries," In *Meth. Enzym.,* 152, 432-442, Academic Press (1987).

Probes and primers can be prepared by enzymatic or recombinant methods, well known to those skilled in the art (*See e.g.* Sambrook et al. *supra*)*.* A probe may be a single stranded nucleic acid sequence which is complementary in some particular degree to a nucleic acid sequence sought to be detected ("target sequence"). A probe may be labeled with a detectable moiety such as a radio-isotope, antigen, or chemiluminescent moiety. A description of the use of nucleic acid hybridization as a procedure for the detection of particular nucleic acid sequences is described in U.S. Patent No. 4,851,330 to Kohne, entitled "Method for Detection, Identification and Quantitation of Non-Viral Organisms."

Having the DNA sequence of the present invention allows preparation of relatively short DNA (or RNA) sequences having the ability to specifically hybridize to nucleic acids that are homologous or identical to sequences disclosed herein. In these aspects, nucleic acid probes of an appropriate length are prepared. The ability of such nucleic acid probes to specifically hybridize to a polynucleotide encoding a Scarface 1 gene or related sequence lends particular utility in a variety of embodiments. Most importantly, the probes may be used in a variety of assays for detecting the presence of complementary sequences or related sequences in a given sample.

In certain embodiments, it is advantageous to use oligonucleotide primers. The sequence of such primers is designed using a polynucleotide of the present invention for use in detecting, amplifying or mutating a defined segment of a gene or polynucleotide that encodes an Scarface 1 polypeptide, using PCR technology.

Preferred nucleic acid sequences employed for hybridization studies, or assays, include probe molecules that are complementary to at least an about 18 to an about 70-nucleotide long stretch of a polynucleotide that encodes an Scarface 1 polypeptide, e.g. SEQ ID NO:1. Molecules having complementary sequences over stretches greater than 18 bases in length are generally preferred, in order to increase stability and selectivity of the hybrid. One will generally prefer to design nucleic acid molecules having gene-complementary stretches of 25 to 40 nucleotides, 55 to 70 nucleotides, or even longer where desired. Such fragments may be readily prepared by, for example, directly synthesizing the fragment by chemical means, by application of nucleic acid reproduction technology, such as the PCR TM technology of U.S. Pat. No. 4,603,102, herein incorporated by reference, or by excising selected DNA fragments from recombinant plasmids containing appropriate inserts and suitable restriction enzyme sites.

The following guidelines are useful for designing probes with desirable characteristics for detecting Scarface 1 homologs. The extent and specificity of hybridization reactions are affected by a number of factors that determine the sensitivity and specificity of a particular probe, whether perfectly complementary to its target or not. The affect of various experimental parameters and conditions are well known to those skilled in the art.

First, the stability of the probe:target nucleic acid hybrid should be chosen to be compatible with the assay conditions. This may be accomplished by avoiding long A and T rich sequences, by terminating the hybrids with G:C base pairs and by designing a probe with an appropriate Tm (i.e. melting temperature). The melting profile, including the Tm of a hybrid comprising an oligonucleotide and target sequence, may be determined using a Hybridization Protection Assay. The probe should be chosen so that the length and % GC content result in a Tm about 2°-10° C higher than the temperature at which the final assay will be performed. The base composition of the probe is also a significant factor because G-C base pairs exhibit greater thermal stability as compared to A-T base pairs. Thus, hybridization involving complementary nucleic acids of higher G-C content will be more stable at higher temperatures.

The ionic strength and incubation temperature under which a probe will be used should also be taken into account. It is known that hybridization will increase as the ionic strength of the reaction mixture increases, and that the thermal stability of molecular hybrids will increase with increasing ionic strength. On the other hand, chemical reagents such as formamide, urea, DMSO and alcohols, which disrupt hydrogen bonds, increase the stringency of hybridization. Destabilization of hydrogen bonds by such reagents can greatly reduce the Tm. In general, optimal hybridization for synthetic oligonucleotide probes of about 10-50 bases in length occurs approximately 5° C below the melting temperature for a given duplex. Incubation at temperatures below the optimum may allow mismatched base sequences to hybridize and can therefore result in reduced specificity.

The length of the target nucleic acid sequence and, accordingly, the length of the probe sequence can also be important. In some cases, there may be several sequences from a particular region, varying in location and length, which will yield probes with the desired hybridization characteristics. In other cases, one sequence may be significantly better than another even though the one sequence differs merely by a single base. Finally, there can be intramolecular and intermolecular hybrids formed within a probe if there is sufficient self-complementarity. Such structures can be avoided through careful probe design. Computer programs are available to search for this type of interaction.

A probe molecule may be used for hybridizing to a sample suspected of possessing a Scarface 1 or Scarface 1 related nucleotide sequence. The hybridization reaction is carried out under suitable conditions of stringency, e.g. high stringency if searching for a closely related sequence, or low stringency if searching for a more distantly related sequence.

Alternatively, such DNA molecules may be used in a number of techniques including their use as: (1) diagnostic tools to detect polymorphisms in DNA samples from a human or other mammal; (2) means for detecting and isolating homologs of Scarface 1 and related polypeptides from a DNA library potentially containing such sequences; (3) primers for hybridizing to related sequences for the purpose of amplifying those sequences; and (4) primers for altering the native Scarface 1 DNA sequences; as well as other techniques which rely on the similarity of the DNA sequences to those of the Scarface 1 DNA segments herein disclosed.

Once synthesized, oligonucleotide probes may be labeled by any of several well known methods. *See e.g.* Maniatis *et.al.,* Molecular Cloning (2d ed. 1989). Useful labels include radioisotopes, as well as non-radioactive reporting groups. Isotopic labels include H³, S³⁵, P³², I¹²⁵, Cobalt, and C¹⁸. Most methods of isotopic labeling involve the use of enzymes and include methods such as nick-translation, end-labeling, second strand synthesis, and reverse transcription. When using radio-labeled probes, hybridization can be detected by autoradiography, scintillation counting, or gamma counting. The detection method selected will depend upon the hybridization conditions and the particular radio isotope used for labeling.

Non-isotopic materials can also be used for labeling, and may be introduced internally into the sequence or at the end of the sequence. Modified nucleotides may be incorporated enzymatically or chemically, and chemical modifications of the probe may be performed during or after synthesis of the probe, for example, by the use of non-nucleotide linker groups. Non-isotopic labels include fluorescent molecules, chemiluminescent molecules, enzymes, cofactors, enzyme substrates, haptens or other ligands. In a preferred embodiment of the invention, the length of an oligonucleotide probe is greater than or equal to about 18 nucleotides and less than or equal to about 50 nucleotides. Labeling of an oligonucleotide of the present invention may be performed enzymatically using [³²P]-labeled ATP and the enzyme T4 polynucleotide kinase.

### In Silico Methods

The invention further comprises in silico methods, or computer-based methods, wherein molecules that are structurally related, or structurally and functionally related to SEQ ID NO:1 and/or SEQ ID NO:2 are identified. In one embodiment, the method comprises comparing SEQ ID NO:1 or SEQ ID NO:2 against a database of nucleic acid sequences, or protein sequences (e.g. GenBank) to identify molecules that are homologous or related to said SEQ ID NO:1 and /or SEQ ID NO:2. For this purpose, the skilled artisan understands that any suitable search or comparison algorithm can be used to identify sequences that are related to SEQ ID NO:1 and/or SEQ ID NO:2. For example, suitable algorithms include dynamic programming algorithms, Hidden Markov Model algorithms, such as for example, the BLAST algorithm, or the Smith-Waterman algorithm, or FASTA.

For example BLAST is used to search for local sequence alignments. BLAST produces alignments of both nucleotide and amino acid sequences. The fundamental unit of the BLAST output is the so-called High-scoring Sequence Pair (HSP). An HSP consists of two sequence fragments of arbitrary but equal lengths whose alignment is locally maximal. BLAST looks for HSPs between a query sequence and a database sequence and retains those comparisons that exceed a user-selected statistical significance threshold. The E parameter establishes the upper bound of the expected frequency of a chance occurrence of an HSP.

For example, a mathematical algorithm utilized for the comparison of two sequences is described in Karlin and Altschul (Proc. Nat. Acad. Sci., 87, 2264, 1990; herein incorporated by reference). Such an algorithm is incorporated in to the NBLAST and XBLAST programs of Altschul et al. J. Mol. Biol., 215, 403 (1990). BLAST nucleotide searches can be performed with the NBLAST program with parameters set to, for example, score = 100, word length = 12, to identify nucleic acid sequences that are homologous, or related to Scarface 1. Protein searches can be performed using the XBLAST program with parameters set to, for example, score = 50, word length = 3, to identify amino acid sequences that are homologous to SEQ ID NO:2. To obtain gapped alignments, Gapped BLAST can be used, as described in Altschul et al. Nuc. Acids Res., 25, 3389 (1997); all of which are herein incorporated by reference. Alternatively, PSI-BLAST can be used to perform an iterated search which detects distant relationships between molecules. If desired, the default parameters may be used in any of the above described algorithms. Alternatively, the ALIGN program, which is part of the GCG sequence alignment software, can be used to detect relationships. When using ALIGN for comparing amino acid sequences, the following parameters may be used: PAM120 weight residue table; gap length penalty 12, and gap penalty 4.

### Vectors

Another aspect of the present invention relates to recombinant DNA cloning vectors and expression vectors comprising the nucleic acids of the present invention. The preferred nucleic acid vectors are those which comprise DNA, for example, SEQ ID NO:1.

The skilled artisan understands that choosing the most appropriate cloning vector or expression vector depends upon a number of factors including the availability of restriction enzyme sites, the type of host cell into which the vector is to be transfected or transformed, the purpose of the transfection or transformation (e.g., stable transformation as an extrachromosomal element, or integration into the host chromosome), the presence or absence of readily assayable or selectable markers (e.g., antibiotic resistance and metabolic markers of one type and another), and the number of copies of the gene desired in the host cell.

Vectors suitable to carry the nucleic acids of the present invention comprise RNA viruses, DNA viruses, lytic bacteriophages, lysogenic bacteriophages, stable bacteriophages, plasmids, viroids, and the like. The most preferred vectors are plasmids.

When preparing an expression vector the skilled artisan understands that there are many variables to be considered, for example, whether to use a constitutive or inducible promoter. The practitioner also understands that the amount of nucleic acid or protein to be produced dictates, in part, the selection of the expression system. Regarding promoter sequences, inducible promoters are preferred because they enable high level, regulatable expression of an operably-linked gene. The skilled artisan will recognize a number of suitable promoters that respond to a variety of inducers, for example, carbon source, metal ions, and heat. Other relevant considerations regarding an expression vector include whether to include sequences for directing the localization of a recombinant protein. For example, a sequence encoding a signal peptide preceding the coding region of a gene is useful for directing the extracellular export of a resulting polypeptide.

The present invention also provides a method for constructing a recombinant host cell capable of expressing the proteins of the invention, said method comprising transforming or otherwise introducing into a host cell a recombinant DNA vector that comprises an isolated DNA sequence that encodes a protein of this invention. A suitable host cell is any eucaryotic cell that can accomodate high level expression of an exogenously introduced gene or protein, and that will incorporate said protein into its membrane structure. Vectors for expression are those which comprise an Scarface 1 gene or fragment thereof, e.g. SEQ ID NO:1. Transformed host cells may be cultured under conditions well known to skilled artisans such that a protein of the present invention is expressed, thereby producing a recombinant Scarface 1 protein in the recombinant host cell.

For the purpose of identifying compounds having utility as modifiers of angiogenesis, it would be desirable to identify compounds that bind an Scarface 1 protein of the present invention, and/or modify its activity. A method for determining agents that bind the Scarface 1 protein and/or inhibit its activity, comprises contacting the Scarface 1 protein with a test compound and monitoring binding by any suitable means.

The instant invention provides a screening system for discovering compounds that bind the Scarface 1 protein, said screening system comprising the steps of:
a) preparing Scarface 1 protein;
b) exposing said Scarface 1 protein to a test compound;
c) quantifying the binding of said compound to Scarface 1 protein by any suitable means, for example, by monitoring a change in the ability of Scarface 1 to phosphorylate a receptor, e.g. TIE 2.

Utilization of the screening system described above provides a means to identify compounds that may alter the biological function of Scarface 1 proteins. This screening method may be adapted to large-scale, automated procedures such as a PANDEX® (Baxter-Dade Diagnostics) system, allowing for efficient high-volume screening for potential therapeutic agents.

In one embodiment, Scarface 1 is prepared as described herein, preferably using recombinant DNA technology. A test compound is introduced into a reaction vessel containing an Scarface 1 protein or fragment thereof. Binding of Scarface 1 by a test compound is determined by any suitable means. For example, in one method radioactively- labeled or chemically-labeled test compound may be used. Binding of the protein by the test compound is assessed, for example, by quantifying bound label versus unbound label using any suitable method.

Binding of a test compound may also be carried out by a method disclosed in U.S. Patent 5,585,277, which hereby is incorporated by reference. In this method, binding of a test compound to a protein is assessed by monitoring the ratio of folded protein to unfolded protein, for example, by monitoring sensitivity of said protein to a protease, or amenability for binding of said protein by a specific antibody against the folded state of the protein.

The foregoing screening methods are useful for identifying an antagonist of a Scarface 1 protein as a lead to a pharmaceutical compound for treating cancer. For example, a compound that binds Scarface 1, or related fragment thereof, is identified by combining a test compound with Scarface 1 under conditions that cause the protein to exist in a ratio of folded to unfolded states. If a test compound binds the folded state of the protein, the relative amount of folded protein will be higher than in the case of a test compound that does not bind the protein. The ratio of protein in the folded versus unfolded state is easily determinable by, for example, susceptibility to digestion by a protease, or binding to a specific antibody, or binding to chaperonin protein, or binding to any suitable surface.

### Therapeutic Applications

In one embodiment, the present invention relates to therapeutic applications in which modulation of angiogenesis is therapeutically beneficial. For example, Scarface 1 can be administered to enhance wound healing such as in post-surgical procedures.

In another embodiment, the invention relates to amplification of stem cells. Amplification of stem cells is useful for a variety of clinically relevant procedures, including, for example, transfusion procedures, or promoting the effectiveness of bone marrow transplants. Because the TIE receptors are expressed in early hematopoietic cells, Scarface 1 is expected to play a comparable role in the proliferation or differentiation or migration of these cells. Thus, for example, TIE containing compositions may be prepared, assayed, examined in in vitro and in vivo biological systems and used therapeutically as described in any of the following: Sousa, U.S. Pat. No. 4,810,643; Lee, et al., Proc. Natl. Acad. Sci. USA 82:4360-4364 (1985); Wong, et al. Science, 228:810-814 (1985); Yokota, et al. Proc. Natl. Acad. Sci (USA) 81:1070 (1984); Bosselman, et al. WO 9105795; and Kirkness, et al. WO 95/19985, herein incorporated by reference.

Accordingly, Scarface 1 may be used to diagnose or treat conditions in which normal hematopoiesis is suppressed, including, but not limited to anemia, thrombocytopenia, leukopenia and granulocytopenia. In a preferred embodiment, Scarface 1 may be used to stimulate differentiation of blood cell precursors in clinical situations where for example a patient has a disease, such as acquired immune deficiency syndrome (AIDS) which has caused a reduction in normal blood cell levels, or in clinical settings in which enhancement of hematopoietic populations is desired, such as in conjunction with bone marrow transplant, or in the treatment of aplasia or myelosuppression caused by radiation, chemical treatment or chemotherapy. Thus, the present invention provides a method for amplifying stem cells, in vivo or in vitro, from a population of cells that contain said stem cells. In one embodiment of this aspect, the invention comprises administering Scarface 1 peptide to amplify hematopoietic stem cells, in vivo or in vitro. For example, stem cell amplification may be achieved in vitro by exposure of bone marrow derived cells to Scarface 1 peptide.

The Scarface 1 of the present invention may be used alone, or in combination with another pharmaceutically active agent such as, for example, ctyokines, neurotrophins, interleukins, etc. In a preferred embodiment, the ligand may be used in conjunction with any of a number of the above referenced factors which are known to induce stem cell or other hematopoietic precursor proliferation, or factors acting on later cells in the hematopoietic pathway, including, but not limited to, hemopoietic maturation factor, thrombopoietin, stem cell factor, erythropoietin, G-CSF, GM-CSF, etc.

The Scarface 1 proteins, peptide fragments, or derivatives may be administered systemically or locally. Any appropriate mode of administration known in the art may be used, including, but not limited to, intravenous, intrathecal, intraarterial, intranasal, oral, subcutaneous, intraperitoneal, or by local injection or surgical implant. Sustained release formulations are also provided for.

The present invention also provides methods for treating cancer and for inhibiting tumor growth, in vitro or in vivo comprising administration of an effective amount of an Scarface 1 antagonist.

For therapeutic use in wound healing or other use in which enhanced angiogenesis is advantageous, an effective amount of Scarface 1 protein is administered to an organism in need thereof in a dose between about 1 and 1000 ug/kg body weight. In practicing the methods contemplated, Scarface 1 can be administered in a single daily dose or in multiple doses per day. The amount per administration will be determined by the physician and depend on such factors as the nature and severity of the disease, and the age and general health of the patient.

The present invention also provides a pharmaceutical composition comprising as the active agent a Scarface 1 polypeptide or fragment, or a pharmaceutically acceptable non-toxic salt thereof, and a pharmaceutically acceptable solid or liquid carrier. For example, compounds comprising Scarface 1 can be admixed with conventional pharmaceutical carriers and excipients, and used in the form of tablets, capsules, elixirs, suspensions, syrups, wafers, and the like. The compositions comprising Scarface 1 will contain from about 0.1% to 90% by weight of the active compound, and more generally from about 10% to 30%. The compositions may contain common carriers and excipients such as corn starch or gelatin, lactose, sucrose, microcrystalline cellulose, kaolin, mannitol, dicalcium phosphate, sodium chloride, and alginic acid. The compounds can be formulated for oral or parenteral administration.

For intravenous (IV) use, the Scarface 1 protein is administered in commonly used intravenous fluid(s) and administered by infusion. Such fluids, for example, physiological saline, Ringer's solution or 5% dextrose solution can be used.

For intramuscular preparations, a sterile formulation, preferably a suitable soluble salt form of the Scarface 1 protein, for example SEQ ID NO:2, such as the hydrochloride salt, can be dissolved and administered in a pharmaceutical diluent such as pyrogen-free water (distilled), physiological saline or 5% glucose solution. A suitable insoluble form of the compound may be prepared and administered as a suspension in an aqueous base or a pharmaceutically acceptable oil base, e.g. an ester of a long chain fatty acid such as ethyl oleate.

Skilled artisans will recognize that IC₅₀ values are dependent on the selectivity of the compound tested. For example, a compound with an IC₅₀ which is less than 10 nM is generally considered an excellent candidate for drug therapy. However, a compound which has a lower affinity, but is selective for a particular target, may be an even better candidate. The skilled artisan will recognize that any information regarding the binding potential, inhibitory activity, or selectivity of a particular compound is useful toward the development of pharmaceutical products.

The following examples more fully describe the present invention. Those skilled in the art will recognize that the particular reagents, equipment, and procedures described are merely illustrative and are not intended to limit the present invention in any manner.

### EXAMPLE 1

### RT-PCR Amplification of Scarface 1 from mRNA

The Scarface 1 gene is isolated by reverse transcriptase PCR (RT-PCR) using conventional methods. Total RNA from a tissue that expresses the Scarface 1 gene, for example, muscle tissue, or reproductive tissues, is prepared using standard methods. First strand cDNA synthesis is achieved using a commercially available kit (SuperScript™ System; Life Technologies) by PCR in conjunction with specific primers directed at any suitable region of SEQ ID NO:1.

Amplification is carried out by adding to the first strand cDNA (dried under vacuum): 8 µl of 10X synthesis buffer (200 mM Tris-HCl, pH 8.4; 500 mM KCl, 25 mM MgCl₂, 1 ug/ul BSA); 68 µl distilled water; 1 µl each of a 10 uM solution of each primer; and 1 µl Taq DNA polymerase (2 to 5 U/µl). The reaction is heated at 94° C for 5 min. to denature the RNA/cDNA hybrid. Then, 15 to 30 cycles of PCR amplification are performed using any suitable thermal cycle apparatus. The amplified sample may be analyzed by agarose gel electrophoresis to check for an appropriately-sized fragment.

### EXAMPLE 2

### Production of a Vector for Expressing Scarface 1 in a Host Cell

An expression vector suitable for expressing Scarface 1 or fragment thereof in a variety of procaryotic host cells, such as *E. coli* is easily made. The vector contains an origin of replication (Ori), an ampicillin resistance gene (Amp) useful for selecting cells which have incorporated the vector following a tranformation procedure, and further comprises the T7 promoter and T7 terminator sequences in operable linkage to a Scarface 1 coding region. Plasmid pET11A (obtained from Novogen, Madison WI) is a suitable parent plasmid. pET11A is linearized by restriction with endonucleases NdeI and BamHI. Linearized pET11A is ligated to a DNA fragment bearing NdeI and BamHI sticky ends and comprising the coding region of the Scarface 1 gene as disclosed by SEQ ID NO:1.

The Scarface 1 gene used in this construction may be slightly modified at the 5' end (amino terminus of encoded protein) in order to simplify purification of the encoded protein product. For this purpose, an oligonucleotide encoding 8 histidine residues is inserted at or near the 5' end of SEQ ID NO:1, so as to maintain the correct reading frame. Placement of the histidine residues at the amino terminus of the encoded protein serves to enable the IMAC one-step protein purification procedure.

### EXAMPLE 3

### Recombinant Expression and Purification of Scarface 1 Protein

An expression vector that carries a gene encoding Scarface 1 or fragment thereof and which gene is operably-linked to an expression promoter is transformed into *E. coli* BL21 (DE3) (*hsdS gal* lcIts857 *ind*1*San*7*nin*5*lac*UV5-T7gene 1) using standard methods. Transformants, selected for resistance to ampicillin, are chosen at random and tested for the presence of the vector by agarose gel electrophoresis using quick plasmid preparations. Colonies which contain the vector are grown in L broth and the protein product encoded by the vector-borne gene is purified by immobilized metal ion affinity chromatography (IMAC), essentially as described in US Patent 4,569,794.

Briefly, the IMAC column is prepared as follows. A metal-free chelating resin (e.g. Sepharose 6B IDA, Pharmacia) is washed in distilled water to remove preservative substances and infused with a suitable metal ion [e.g. Ni(II), Co(II), or Cu(II)] by adding a 50mM metal chloride or metal sulfate aqueous solution until about 75% of the interstitial spaces of the resin are saturated with colored metal ion. The column is then ready to receive a crude cellular extract containing the recombinant protein product.

After removing unbound proteins and other materials by washing the column with any suitable buffer, pH 7.5, the bound protein is eluted in any suitable buffer at pH 4.3, or preferably with an imidizole-containing buffer at pH 7.5.

### EXAMPLE 4

### Tissue Distributuion of Scarface 1 mRNA

Scarface 1 mRNA was detected in a mammalian tissue by Northern analysis. Total RNA from different tissues was isolated by a standard guanidine chloride/phenol extraction method, and poly-A⁺ RNA isolated using oligo(dT)-cellulose type 7 (Pharmacia). Electrophoresis of RNA samples was carried out in formaldehyde followed by capillary transfer to Zeta-Probe™ nylon membranes (Bio-Rad, Hercules, Calif.). SEQ ID NO:1 was used as a template for generating probes using a MultiPrime™ random priming kit (Amersham, Arlington Heights, Ill.). The efficiency of the labeling reaction was approximately 4 x 10¹⁰ cpm incorporated per µg of template. The hybridization buffer contained 0.5M sodium phosphate, 7% SDS (wt/vol), 1% BSA (wt/vol), and 1 mM EDTA. Prehybridization was carried out in hybridization buffer at 65° C for 2 h, and ³²P-labeled probe was added and incubation continued overnight. Filters were washed in Buffer A (40 mM sodium phosphate pH 7.2, 5% SDS [wt/vol], 0.5% BSA [wt/vol], and 1 mM EDTA) at 65° C for 1 h, then in Buffer B (40 mM sodium phosphate, pH 7.2, 1% SDS [wt/vol], and 1 mM EDTA) at 65° C for 20 min. Washed filters were air-dried and exposed to Kodak X-OMAT AR film at -80° C with an intensifying screen. The results (see Figure 1) demonstrate that Scarface 1 mRNA is expressed in a plurality of tissues and that two major species are observed, one approximately 2.4 Kb in size and the other about 4 Kb.

### EXAMPLE 5

### Detecting Ligands that Bind Scarface 1 Using a Chaperonin Protein Assay

The wells of an ELISA plate are coated with chaperonin by incubation for several hours with a 4 ug/ml solution of the protein in Tris-buffered Saline (TBS: 10 mM Tris-HCl, pH7.5, 0.2M NaCl). Plates are washed 3 times with TBS containing 0.1% Tween-20 (TBST). Then, a mixture of Scarface 1 protein (sufficient amount to saturate about 50% of the binding sites on chaperonin) and test compound (10⁻⁹ to 10⁻⁵ M) in about 50 µl volume is added to each well of the plate for an incubation of about 60 minutes. Aliquots from each well are then transferred to the wells of fresh plates and incubation is continued for 60 minutes at room temperature, followed by 3 washes with TBST. Next, about 50 µl of an antibody specific for Scarface 1 in 5% nonfat dry milk is added to each well for 30 minutes at room temperature. After washing, about 50 µl of goat anti-rabbit IgG alkaline phosphatase conjugate at an appropriate dilution in TBST plus 5% nonfat dry milk is added to each will and incubated 30 minutes at room temperature. The plates are washed again with TBST, and 0.1 ml of 1 mg/ml p-nitrophenylphosphate in 0.1% diethanolamine is added. Color development (proportional to bound alkaline phosphatase antibody conjugate) is monitored with an ELISA plate reader. When binding by the test ligand has occurred, ELISA analysis reveals Scarface 1 in solution at higher concentrations than in the absence of test ligand.

### EXAMPLE 6

### Production of an Antibody to Scarface 1 Protein

Substantially pure Scarface 1 protein or fragment thereof or analog thereof is isolated from transfected or transformed cells using any of the well known methods in the art, or by a method specifically disclosed herein. Concentration of protein is adjusted, for example, by filtration through an Amicon filter device such that the level is about 1 to 5 ug/ml. Monoclonal or polyclonal antibody is prepared as follows.

Monoclonal antibody can be prepared from murine hybridomas according to the method of Kohler and Milstein (*Nature,* 256, 495, 1975), or a modified method thereof. Briefly, a mouse is repetitively inoculated with a few micrograms of the Scarface 1 protein (e.g. SEQ ID NO:2) or fragment thereof, or analog thereof, over a period of a few weeks. The mouse is then sacrificed, and antibody producing cells of the spleen are isolated. Spleen cells are fused with mouse myeloma cells using polyethylene glycol. Fused cells that produce antibody specific for Scarface 1 are identified by any suitable immunoassay, for example, ELISA, as described in E. Engvall, *Meth. Enzymol.,* 70, 419, 1980.

Polyclonal antiserum can be prepared by well known methods (*See e.g.* J. Vaitukaitis *et.al. Clin. Endocirnol. Metab.* 33, 988, 1971) that involve immunizing suitable animals with a Scarface 1 protein, fragments thereof, or fusion proteins thereof. Small doses (e.g. nanogram amounts) of antigen administered at multiple intradermal sites are the most reliable method.

### EXAMPLE 7

### Assay For Scarface 1 Activity

Scarface 1 activity is assayed by measuring tyrosine-specific phosphorylation, essentially as described in S. Davis *et al., Cell,* 87, 1161-1169, 1996; and T. Stitt *et. al. Cell,* 80, 661-670, 1995, both of which are herein incorporated by reference. Briefly, assays are done using late-confluency T-75 flasks of human umbilical vein endothelial cells (available from Clonetics, Inc. San Diego, CA). Cells are serum starved in high glucose DMDM for 2 - 4 hr. Conditioned medium, plus and minus varying amounts of Scarface 1 protein from about 10 ng/ml to about 10 ug/ml, is placed on cells for 5 - 10 min. at 37°C. Cells are then lysed in 1% NP-40 in PBS containing 1 mM PMSF, 0.14 U/ml aprotinin, 1 mM EDTA, and 1 mM sodium orthovanadate. The lysates are immunoprecipitated with a phosphotyrosine specific rabbit antiserum, electrophoresed, and immunoblotted (Western Blot) to nylon membranes. Following incubation of the blots with HRP-conjugated goat anti-rabbit antibodies ( Caltag, Inc.) bands are visualized by ECL.

### EXAMPLE 8

### Assay For Scarface 1 Antagonist

Assay reactions are set up essentially as described in Example 7, accept that a compound to be tested for Scarface 1 antagonist activity is included at the step of adding Scarface 1 to conditioned medium for 5 - 10 min. prior to cell lysis. Multiple assays can be set up in which a constant amount of Scarface 1 is incubated with varying amounts of test compound, for example from about 10 ng/ml to about 100 ug/ml.

## Claims

1. A substantially pure protein having an amino acid sequence that is encoded by a nucleic acid sequence that hybridizes to SEQ ID NO:1 under high stringency conditions said protein having Scarface 1 activity.

2. A substantially pure Scarface 1 protein having an amino acid sequence identified herein as SEQ ID NO: 2.

3. A functional fragment of a Scarface 1 protein or analog comprising a fibrinogen-like domain selected from the group consisting of:
a) a domain having an amino acid sequence that is structurally and functionally related to residues 275 through 493 of SEQ ID NO:2; and
b) a domain having an amino acid sequence that is defined by residues 275 through 493 of SEQ ID NO:2.

4. An isolated nucleic acid compound encoding a protein of Claim 1, Claim 2, or Claim 3.

5. An isolated nucleic acid sequence that encodes Scarface 1 protein wherein said nucleic acid is SEQ ID NO:1.

6. A vector comprising an isolated nucleic acid of Claim 4 or Claim 5.

7. A pharmaceutical formulation comprising as an active ingredient an Scarface 1 protein or analog thereof, associated with one or more pharmaceutically acceptable carriers, excipients, or diluents thereof.

8. A host cell containing a vector of Claim 6.

9. A method for identifying an Scarface 1 antagonist or agonist comprising the steps of:
a) admixing a substantially purified preparation of Scarface 1 with a test compound; and
b) monitoring, by any suitable means, an inhibition or enhancement respectively in the biological activity of Scarface 1.

10. A method for expanding hematopoietic stem cells comprising the step of administration of Scarface 1 to a population of bone marrow cells.
